# EUROPEAN PATENT APPLICATION

(11) **EP 2 368 481 A1**
(43) Date of publication of application: **28.09.2011**
(21) Application number: 11152074.8
(22) Date of filing: 25.01.2011
(51) Int. Cl.: A61B 1/01, A61B 1/015, A61B 1/31, A61B 17/34, A61M 25/06, A61M 25/10

(54) **Guide device for endoscope**

(30) Priority: 25.03.2010 JP 2010069309
(71) Applicant: Fujifilm Corporation, Minato-ku Tokyo (JP)
(72) Inventor: Yamamoto, Seiichi, Kanagawa (JP); Ueda, Yoshihiro, Kanagawa (JP)
(74) Representative: Klunker . Schmitt-Nilson . Hirsch

(57) **Abstract**

A guide device for an endoscope (10) as balloon tube assembly includes an elongated tube (61) having a tube lumen (70) for receiving entry of an insertion tube (13) of the endoscope, for entry in a body cavity together with the endoscope. A collection channel (72, 121) is formed inside the elongated tube together with the tube lumen, for collecting body fluid present between the insertion tube and a surface of the tube lumen. A profile of the elongated tube is circular when viewed in a cross section. A combination of the collection channel and the tube lumen within the elongated tube is substantially in a keyhole shape when viewed in a cross section. Furthermore, a proximal end handle (60) constitutes one end of the elongated tube with an inclination toward a side opposite to a side where the collection channel is disposed. A balloon (78) is provided at a distal end of the elongated tube.

## Description

### BACKGROUND OF THE INVENTION

### 1. Field of the Invention

The present invention relates to a guide device for endoscope. More particularly, the present invention relates to a guide device which is used for guiding entry of an endoscope in a body cavity, and can have a high durability and operability and a simple structure without an unwanted large diameter.

### 2. Description Related to the Prior Art

An endoscope is used for medical imaging of a body cavity of a patient, and has an insertion tube or elongated tube. For diagnosis and treatment, the insertion tube is entered in a gastrointestinal tract as deeply as an intestine and colon. The intestine and colon are considerably tortuous. Entry of the insertion tube of the endoscope in the intestine and colon is highly difficult, because force of its manipulation of a doctor or operator is difficult to transmit to a distal end of the insertion tube. There is a known method of using an overtube or sliding tool or guide device. The insertion tube of the endoscope is inserted in a tube lumen of the overtube, and is entered into a body cavity together with the overtube. The overtube can guide the insertion tube of the endoscope, and thus makes it possible to manipulate the insertion tube to reach a deep site in the gastrointestinal tract, because occurrence of an unwanted bend or tortuous form of the insertion tube can be prevented.

In the manipulation of the overtube or guide device, there occurs a problem in that body fluid flows back through a clearance space between the insertion tube of the endoscope and a surface of the tube lumen of the overtube, to leak the body fluid to the outside through a proximal end portion of the overtube. In JP-A 2005-312905 (corresponding to U.S. Pat.Pub. Nos. 2005/124856 and 2007/299308), a collection channel for fluid is formed in the proximal end portion of the overtube to solve such a problem.

In JP-A 2005-312905, various examples of the collection channel are disclosed, including a spherical shape with which the proximal end portion is locally protruded, and a bellows shape or bag shape expandable in a longitudinal axis direction. To prevent leaking of body fluid to the outside, a number of structures are suggested, including a sponge or porous material disposed in the collection channel of a spherical shape for absorbing fluid, a combination of packing and an annular retaining device disposed in the proximal end portion, and an anti-backflow valve constituted by a bag end of the collection channel of the bag shape.

The examples of the collection channel in JP-A 2005-312905, in such shapes as a sphere, bellows, a bag and the like, may increase a diameter of the overtube or guide device. Kink, buckling or other unwanted deformation is likely to occur at the collection channel, so that durability and operability will be rather low. Although the use of a multi-lumen tube may be effective for producing a tube for discharging body fluid, there occurs another problem of a considerably high cost of manufacture.

### SUMMARY OF THE INVENTION

In view of the foregoing problems, an object of the present invention is to provide a guide device which is used for guiding entry of an endoscope in a body cavity, and can have a high durability and operability and a simple structure without an unwanted large diameter.

In order to achieve the above and other objects and advantages of this invention, a guide device for an endoscope includes an elongated tube having a tube lumen for receiving entry of an insertion tube of the endoscope, for entry in a body cavity together with the endoscope. A collection channel is formed inside the elongated tube together with the tube lumen, for collecting body fluid present between the insertion tube of the endoscope and an inner surface of the tube lumen. A profile of the elongated tube is substantially circular when viewed in a cross section, and a combination of the collection channel and the tube lumen within the elongated tube is substantially in a keyhole shape when viewed in a cross section.

Furthermore, a proximal opening is positioned at a proximal end of the tube lumen, and adapted to exhaust of air from the collection channel.

Furthermore, an inclined surface is formed inside the proximal opening, and inclined to increase openness thereof outwards.

Furthermore, a fluid opening is formed in an outer surface of the elongated tube at the collection channel, for intake of the body fluid.

Furthermore, at least one through lumen is formed in a wall of the elongated tube beside the collection channel discretely from the tube lumen.

The at least one through lumen is constituted by two lumens between which the tube lumen is disposed.

The collection channel extends substantially at a full length of the elongated tube, and is used for receiving entry of a second endoscope or treatment instrument additionally to the tube lumen.

In one preferred embodiment, furthermore, a proximal end tube constitutes one end of the elongated tube. The collection channel is at least disposed adjacently to a distal side of the proximal end tube.

Furthermore, a proximal end tube constitutes one end of the elongated tube with an inclination toward a side opposite to a side where the collection channel is disposed.

Furthermore, a balloon is provided at a distal end of the elongated tube.

Furthermore, an inflation opening is formed in the elongated tube, disposed within the balloon. The through lumen communicates with the inflation opening, and expands and compresses the balloon with air.

The guide device is a balloon tube assembly.

Also, an endoscope system is provided, and includes an endoscope and a guide device for guiding the endoscope. The guide device includes an elongated tube, having a tube lumen for receiving entry of an insertion tube of the endoscope, for entry in a body cavity together with the endoscope. A collection channel is formed inside the elongated tube together with the tube lumen, for collecting body fluid present between the insertion tube of the endoscope and an inner surface of the tube lumen. A profile of the elongated tube is substantially circular when viewed in a cross section, and a combination of the collection channel and the tube lumen within the elongated tube is substantially in a keyhole shape when viewed in a cross section.

Consequently, a guide device can have a high durability and operability and a simple structure without an unwanted large diameter, because of the structure of the collection channel in connection with the tube lumen.

### BRIEF DESCRIPTION OF THE DRAWINGS

The above objects and advantages of the present invention will become more apparent from the following detailed description when read in connection with the accompanying drawings, in which:
Fig. 1 is an explanatory view illustrating an endoscope system;
Fig. 2 is a front elevation illustrating an end of a head assembly of an endoscope;
Fig. 3 is a cross section illustrating a balloon tube assembly having an overtube and a balloon;
Fig. 4 is a vertical section illustrating a distal end portion of the balloon tube assembly;
Fig. 5 is a cross section illustrating one preferred balloon tube assembly having additional tube lumens;
Fig. 6 is a side elevation illustrating another preferred balloon tube assembly in which a collection channel extends at a full length.

### DETAILED DESCRIPTION OF THE PREFERRED EMBODIMENT(S) OF THE PRESENT INVENTION

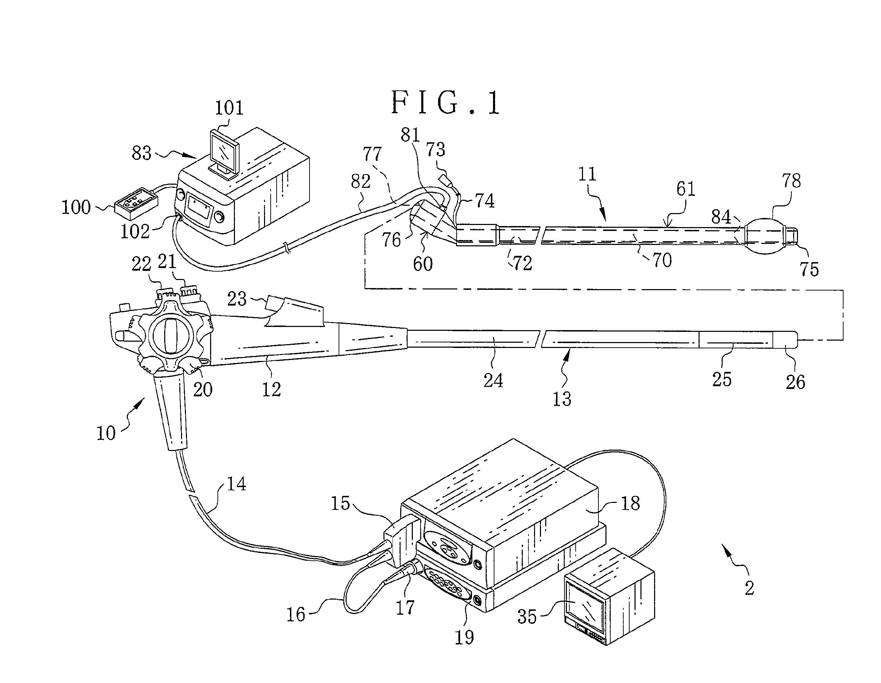

In Fig. 1, an endoscope system 2 includes an electronic endoscope 10 and a balloon tube assembly 11 or overtube assembly or outer tube assembly as guide device. The endoscope 10 includes a handle 12 and an elongated tube 13 or insertion tube which extends from the handle 12 and is used for entry in a gastrointestinal tract as a body cavity, for example, large intestine. A universal cable 14 is connected to the handle 12. A light source connector 15 or plug is disposed at an end of the universal cable 14. A cable 16 extends from an end of the light source connector 15 as a branch. A connector 17 or plug is disposed at an end of the cable 16. A light source apparatus 18 is connected by use of the light source connector 15. A processing apparatus 19 is connected by use of the connector 17.

The handle 12 includes a steering wheel 20, an air/water supply button 21 and a suction button 22. The air/water supply button 21 is operated to supply air or water through a distal end of the insertion tube 13. A forceps port 23 is formed through the handle 12 near to the insertion tube 13 for entry of an electrosurgical instrument, forceps or other medical instrument for treatment.

The insertion tube 13 includes a flexible unit 24, a steering device 25 and a head assembly 26 in an order of a distal direction on the handle 12. The flexible unit 24 is as long as several meters for reach of the head assembly 26 to an object of interest in a body cavity. The steering device 25 is steered with bends in upward and downward directions and directions to the right and left by rotational operation of the steering wheel 20 at the handle 12. Thus, it is possible to direct the head assembly 26 in any direction toward an intended point in the body cavity.

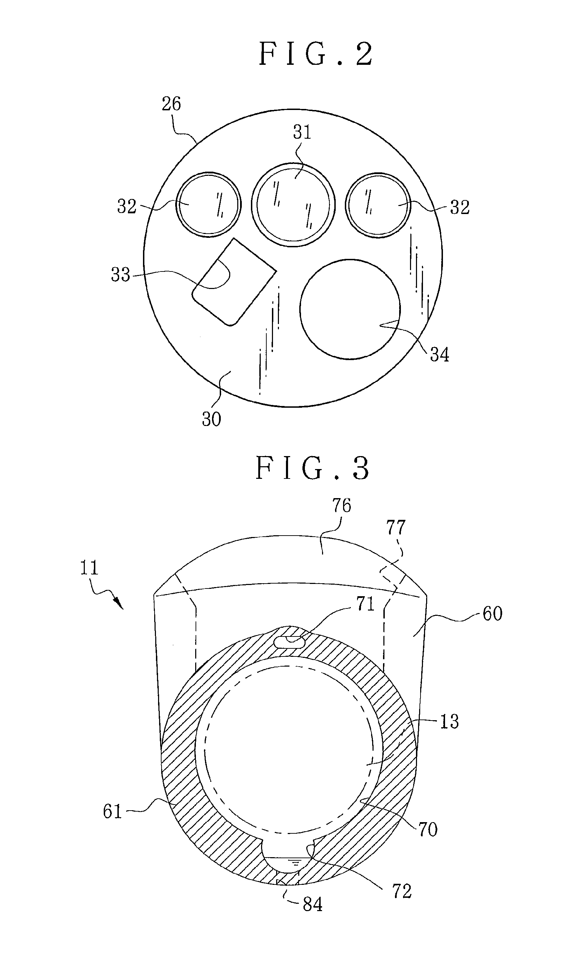

In Fig. 2, there is a distal end surface 30 of the head assembly 26. Various openings are formed in the distal end surface 30, including an imaging window 31, lighting windows 32, a spray nozzle 33 or supply nozzle, and a forceps opening 34. The imaging window 31 is disposed at the center on one side of the distal end surface 30. The lighting windows 32 are two disposed symmetrically with respect to the imaging window 31.

Elements are disposed inside the imaging window 31, including an objective lens system and an image pickup device. The objective lens system receives image light from an object in a body cavity. Examples of the image pickup device are a CCD, CMOS image sensor and the like for picking up the object image. A signal cable is disposed to extend from the image pickup device, is entered through the insertion tube 13, the handle 12 and the universal cable 14 to the connector 17, and connects the image pickup device to the processing apparatus 19. The object image is focused on an image pickup surface of the image pickup device after passage of light through the imaging window 31, and is converted into an image signal. The processing apparatus 19 processes the image signal in the image processing of various functions into a video signal after transmission of the image signal through the signal cable from the image pickup device. A monitor display panel 35 is caused to display an image according to the video signal. See Fig. 1.

An exit end of a light guide device is disposed behind the lighting windows 32 for guiding light from the light source apparatus 18. The light guide device extends through the insertion tube 13, the handle 12 and the universal cable 14 toward the light source connector 15, in which its entrance end is contained. The light guided by the light guide device is applied to an object of interest in a body cavity through the lighting windows 32.

The spray nozzle 33 supplies air or water from an air/water supply device in the light source apparatus 18 toward the imaging window 31 in response to depression of the air/water supply button 21. A forceps channel (not shown) is formed in the insertion tube 13. The forceps opening 34 is connected to the forceps channel extending toward the forceps port 23. A distal end of a medical instrument entered through the forceps port 23 emerges from the forceps opening 34.

In Fig. 1, the balloon tube assembly 11 includes a proximal end handle 60 or proximal end tube, and an elongated tube or insertion tube 61. The proximal end handle 60 is a rigid part of a barrel shape formed from plastic material or the like. The elongated tube 61 is formed from flexible material such as polyurethane, and is secured firmly to a distal end of the proximal end handle 60. A collection channel 72 for fluid is formed through the elongated tube 61. The proximal end handle 60 extends with a bend at a suitable angle in an upward direction which is opposite to a downward side on which the collection channel 72 is located.

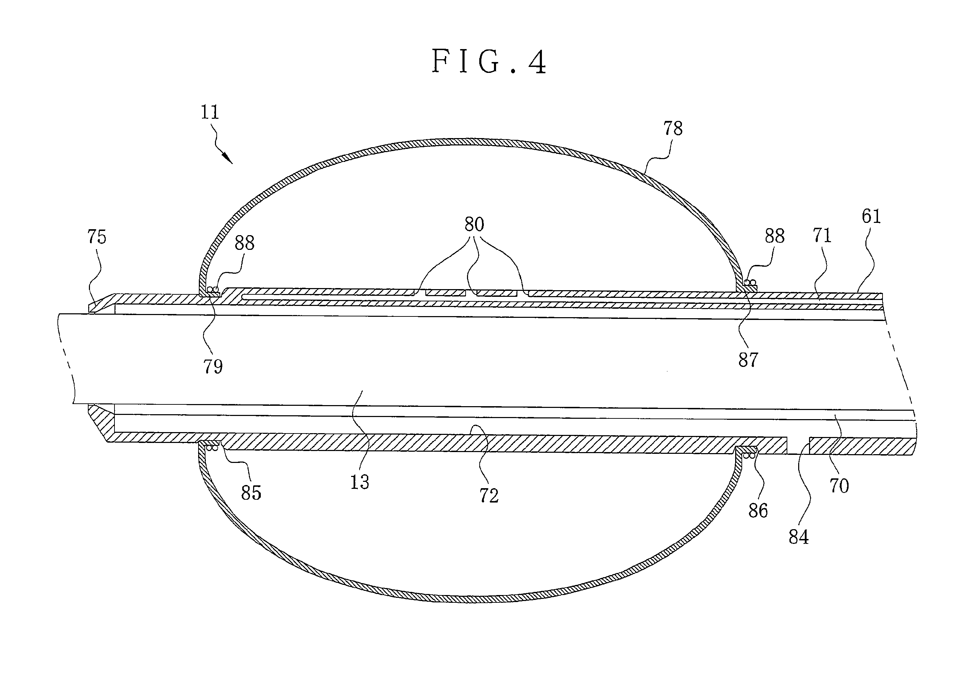

In Figs. 3 and 4, a tube lumen 70 and an inflation lumen 71 or through lumen are formed through the elongated tube 61 axially and together with the collection channel 72. The tube lumen 70 receives entry of the insertion tube 13 of the endoscope 10. As viewed in a cross section of the elongated tube 61, the tube lumen 70 is circular except for a portion in connection with the collection channel 72. An inner diameter of the tube lumen 70 is slightly greater than an outer diameter of the insertion tube 13. For example, the outer diameter of the insertion tube 13 is 10 mm. The tube lumen 70 has such a diameter that a clearance space of approximately 0.7 mm is defined from its inner surface to the insertion tube 13. The inner surface of the tube lumen 70 is coated with hydrophilic coating agent or lubricant coating agent, for example, polyvinyl pyrrolidone.

To use the balloon tube assembly 11, lubricating fluid such as water is supplied to the inner surface of the tube lumen 70 or clearance space between the insertion tube 13 and the elongated tube 61, to reduce friction between the insertion tube 13 and the elongated tube 61. There is a tube connector 73 in Fig. 1. A syringe (not shown) is used to inject the lubricating fluid through the tube connector 73. A flow tube 74 of a small bore has a proximal end connected to the tube connector 73, and has a distal end connected to a proximal end of the tube lumen 70. The lubricating fluid injected in the tube connector 73 is supplied to the inner surface of the tube lumen 70 through the flow tube 74.

An inclined wall 75 is formed at a distal end of the elongated tube 61 for decreasing the inner diameter. See Fig. 1. When the insertion tube 13 of the endoscope 10 is entered in the tube lumen 70, the inclined wall 75 reduces a gap between the insertion tube 13 and the distal end of the elongated tube 61, to prevent leakage of the lubricating fluid in a distal direction of the elongated tube 61. An inlet tube 76 as exhaust hole of rubber or other elastic material is disposed on a proximal end of the balloon tube assembly 11 (or of the proximal end handle 60). See Fig. 1. A funnel-shaped proximal opening 77 as exhaust hole is formed in a proximal end portion of the inlet tube 76, and has an inner diameter greater than that of the tube lumen 70. The tube lumen 70 and the collection channel 72 communicate to the outside by the funnel-shaped proximal opening 77. Note that the inclined wall 75 can be formed in an entire peripheral surface of a distal end of the elongated tube 61, or only in a part of the entire peripheral surface.

There is a balloon 78, for which the inflation lumen 71 supplies or draws fluid, such as air. The inflation lumen 71 extends through a wall of the tube lumen 70. As viewed in a cross section of the elongated tube 61, the inflation lumen 71 is in a shape of an ellipse of which a long axis extends in a direction along an arc of the elongated tube 61 and a short axis extends in its radial direction. It is possible to shape the elongated tube 61 without protrusion of a portion of the inflation lumen 71 in an outward direction, and to keep a sufficient area of a flow path in the inflation lumen 71. Note that the inflation lumen 71 can be shaped in other forms having a long axis extending in a direction along an arc of the elongated tube 61 and a short axis extending in its radial direction. For example, the inflation lumen 71 can be shaped in a form curved along a circumference to the tube lumen 70 as viewed in a cross section.

A distal balloon end 79 is a portion of the balloon 78. An end of the inflation lumen 71 is closed at a fixed point of the distal balloon end 79. Inflation openings 80 are formed in the outer surface of the elongated tube 61. The inflation lumen 71 extends to communicate with the inflation openings 80. Annular grooves 85 and 86 are formed in the elongated tube 61. The inflation openings 80 are arranged between the annular grooves 85 and 86 or disposed for positioning the balloon 78. Air is drawn in and out through the inflation openings 80 to expand and compress the balloon 78.

The collection channel 72 collects body fluid by gas-liquid separation of the body fluid and bubbles present together between the insertion tube 13 of the endoscope 10 and an inner surface of the tube lumen 70. The collection channel 72 is formed together with the tube lumen 70, and located on a side opposite to a direction of the bend of the proximal end handle 60. As viewed in the cross section of the elongated tube 61, the collection channel 72 is defined with an arc extending from points on the periphery of the tube lumen 70. A combination of the tube lumen 70 and the collection channel 72 has a gourd shape or keyhole shape as viewed in the cross section. A diameter of the arc defining the collection channel 72 is in a range of 3-8 mm.

A distal end of the collection channel 72 is closed at a point short of the inclined wall 75. A proximal end of the collection channel 72 is closed at a proximal end of the elongated tube 61 for junction with the proximal end handle 60. See Fig. 1. The collection channel 72 communicates with the outside through the funnel-shaped proximal opening 77 of the inlet tube 76 together with the tube lumen 70.

In Fig. 1, a tube connector 81 is disposed at the proximal end handle 60. A proximal end of the inflation lumen 71 communicates with the tube connector 81. There is a balloon controller 83. A flow tube 82 is connected between the tube connector 81 and the balloon controller 83. The balloon controller 83 supplies or draws air to expand or compress the balloon 78. Note that a tube of a small bore may be connected to a proximal end of the inflation lumen 71. The tube connector 81 may be disposed at an end of this tube, which is similar to the combination of the tube connector 73 and the flow tube 74.

In Fig. 4, a fluid opening 84 is formed in the elongated tube 61, positioned on a proximal side from the balloon 78, for communication from the collection channel 72 to the outside. Air and body fluid or mucus from the body flows through the fluid opening 84 into the clearance space between the insertion tube 13 of the endoscope 10 and the inner surface of the tube lumen 70.

The annular grooves 85 and 86 are formed in an outer surface of a distal portion of the elongated tube 61 for mounting the balloon 78. The annular groove 85 extends in a full circumference of the outer surface. The annular groove 86 extends in a C shape and except for the vicinity of the inflation lumen 71. The distal balloon end 79 is fitted in the annular groove 85. A proximal balloon end 87 of the balloon 78 is fitted in the annular groove 86.

The balloon 78 has a roll shape with a central projecting portion. Initially, the balloon 78 is conditioned in a state inside out, and set on the elongated tube 61 by disposing the distal balloon end 79 in the annular groove 85 of the elongated tube 61. A string 88 or strip is wound about the distal balloon end 79 of the balloon 78. A coating of adhesive agent is applied to the string 88, to fix the distal balloon end 79 on the elongated tube 61. Then the balloon 78 is returned to an original state before being inside out. The proximal balloon end 87 of the balloon 78 is disposed in the annular groove 86. The string 88 is wound about the proximal balloon end 87 of the balloon 78. A coating of adhesive agent is applied to the string 88 to fix the proximal balloon end 87 on the elongated tube 61. Finally, the distal and proximal balloon ends 79 and 87 of the balloon 78 are firmly secured to the annular grooves 85 and 86. The balloon 78 can be mounted without projecting its fixing portion to the outside, because the distal and proximal balloon ends 79 and 87 are positioned at the annular grooves 85 and 86.

The elongated tube 61 is produced by working a multi-lumen tube of which a sectional form is constant. Before working, the multi-lumen tube has three lumens which penetrate axially and will be the tube lumen 70, the collection channel 72 and the inflation lumen 71 after the working. The sectional form of the multi-lumen tube remains unchanged. In the multi-lumen tube, a core of metal is inserted. A mold of a barrel shape for press, having two ridges on its inner surface, is pressed to the multi-lumen tube, which is heated at a predetermined temperature, for example 100-110 deg. C. Thus, the annular grooves 85 and 86 are formed in the multi-lumen tube finally to produce the elongated tube 61. Note that an initial form of the multi-lumen tube has the three lumens. To obtain the final form of the elongated tube 61, a wall portion present between two of the three lumens is cut by a suitable blade so that a single combined lumen of a non-cylindrical form is obtained inclusive of the tube lumen 70 and the collection channel 72.

In Fig. 1, the balloon controller 83 causes air or other fluid to flow into and out of the balloon 78. A control panel or switch device 100 and a balloon monitor display panel 101 are associated with the balloon controller 83.

A front surface of the balloon controller 83 has a power switch, a stop switch, a pressure indicator and the like. The pressure indicator is a display panel for indicating a pressure value of the balloon 78. Upon occurrence of failure, such as breakage of the balloon 78, the pressure indicator indicates an error code.

An anti-backflow device 102 or valve is provided at a point of connection between the balloon controller 83 and the flow tube 82 for the balloon 78. The anti-backflow device 102 is constituted by a gas-liquid separation filter incorporated in a case of a disk shape mounted on the balloon controller 83 removably. If the balloon 78 is broken, the anti-backflow device 102 prevents the fluid such as body fluid or mucus from flowing into the balloon controller 83.

Various switches are included in the control panel 100. Among those, a stop switch is disposed to operate in the same manner as that for the balloon controller 83. A start switch turns on and off for command of compressing and decompressing the balloon 78. A pause switch keeps an inner pressure of the balloon 78. A cable electrically connects the control panel 100 to the balloon controller 83. An indicator (not shown) is disposed on the control panel 100 for indicating conditions of air supply and air discharge of the balloon 78.

The balloon controller 83 supplies air to the balloon 78 for expansion, and also controls pressure of the air to keep the balloon 78 expanded. Also, the balloon controller 83 operates for suction of air from the balloon 78 for compressing, and controls the air pressure at a constant level for keeping the balloon 78 compressed.

The balloon monitor display panel 101 displays various data in operation of expanding and compressing the balloon 78, such as a pressure value, states of the expansion and compression. Note that those data on the display panel 35 can be superimposed visibly on an object image of a body part viewed in the endoscope 10.

The operation of manipulating the endoscope system 2 is described now. The insertion tube 13 of the endoscope 10 and the balloon tube assembly 11 are entered into a body cavity alternately in a push method. The balloon 78 is expanded when required, to anchor the balloon tube assembly 11. Then the balloon tube assembly 11 is pulled in a proximal direction of the proximal end handle 60 to simplify a form of the balloon tube assembly 11 in the body cavity. Namely, a tortuous form of the balloon tube assembly 11 is locally eliminated. Then the insertion tube 13 is entered further with advance in the body cavity. A doctor or operator manipulates the endoscope system 2 by orienting the bend of the proximal end handle 60 upwards.

Body fluid and bubbles (air component present behind the balloon 78), introduced through the fluid opening 84 and present between the inner surface of the tube lumen 70 and the insertion tube 13 of the endoscope 10, are separated by operation of a relatively large clearance space between the insertion tube 13 and the collection channel 72. Should the collection channel 72 not exist, a clearance space between the insertion tube 13 and the inner surface of the tube lumen 70 is as small as 0.7 mm because an inner diameter of the tube lumen 70 is slightly greater than an outer diameter of the insertion tube 13. Mixture of the body fluid and bubbles flows in the clearance space. In contrast, forming of the collection channel 72 makes it possible to keep a relatively large clearance space to the insertion tube 13. Bubbles can be separated from the body fluid by the clearance space. The body fluid is collected in the collection channel 72. Air of the bubbles is drawn through the tube lumen 70 and the collection channel 72, and discharged to the outside through the funnel-shaped proximal opening 77 of the inlet tube 76.

In movement of the balloon tube assembly 11 in a proximal direction in a condition of expanding the balloon 78, the tube is pulled toward the outside. A component of air present on a proximal side of the balloon 78 is compressed to have a raised air pressure, which may be resistant to the movement of the balloon tube assembly 11 in the proximal direction. However, the air component flows into the clearance space between the insertion tube 13 of the endoscope 10 and the inner surface of the tube lumen 70 through the fluid opening 84, is separated from a liquid component by the collection channel 72, and is discharged through the funnel-shaped proximal opening 77. This is effective in preventing an increase in the air pressure of the air component behind the balloon 78. The balloon tube assembly 11 can be smoothly pulled in the proximal direction.

The body fluid collected in the collection channel 72 is discharged by orienting the balloon tube assembly 11 upside down or in a direction reverse to that during the manipulation, and drained to a waste receptacle by directing down the bend of the proximal end handle 60.

As the collection channel 72 is formed together with the tube lumen 70, and their combination has a keyhole shape or gourd shape as viewed in a cross section, a required space for the collection channel 72 can be minimized. The balloon tube assembly 11 can have a reduced diameter in comparison with that with a collection channel having a spherical shape, bellows shape or bag shape.

For the gas-liquid separation between the body fluid and bubbles present between the insertion tube 13 and the inner surface of the tube lumen 70, it may be possible to enlarge the clearance space between those, or to enlarge an inner diameter of the tube lumen 70 over an outer diameter of the insertion tube 13. However, enlargement of the inner diameter of the tube lumen 70 will cause a problem of reducing possibility of a decrease in the diameter of the balloon tube assembly 11, and also is likely to create kink or unwanted deformation of a portion of the tube lumen 70 with a greater inner diameter. The simple enlargement of the inner diameter of the tube lumen 70 is not favorable in view of good durability and operability. In contrast, use of a multi-lumen tube for constructing a channel for draining body fluid will raise a manufacturing cost considerably.

As described heretofore, the collection channel 72 is formed together with the tube lumen 70. Their combined form has a keyhole shape or gourd shape as viewed in the cross section of the elongated tube 61. An outer surface of the elongated tube 61 is circular as viewed in the cross section. This is effective in keeping durability and operability without kink, and solving the problem of a high manufacturing cost due to a form having a channel for discharging body fluid or mucus.

The proximal end handle 60 is bent in a direction opposite to a side of disposing the collection channel 72. When the elongated tube 61 is manipulated by upwards directing the bend of the proximal end handle 60, a position of the funnel-shaped proximal opening 77 can be kept higher constantly than a level of the body fluid present in the collection channel 72. See Fig. 3. Accordingly, the bend of the proximal end handle 60 as a remarkably simple structure can prevent leakage of body fluid from the collection channel 72. It is unnecessary to use a sponge for water absorption, to use a packing with an annular retaining device, or to use a bag end portion as an anti-backflow valve.

Also, the proximal end handle 60 can be shaped straight without a bend. In a manner similar to the inclined wall 75, the inlet tube 76 has a diameter decreasing in the proximal direction, to prevent leakage of lubricating fluid to the outside. Another exhaust opening is formed for communication with the tube lumen 70 in the proximal end handle 60 in place of the funnel-shaped proximal opening 77.

In the above embodiments, the inflation lumen 71 is located on a side opposite to that of the collection channel 72. However, the inflation lumen 71 may be located close to the collection channel 72. In Fig. 3, a wall of the elongated tube 61 is locally thicker in the vicinity of the collection channel 72 than a remaining part of the elongated tube 61. It is possible to reduce the diameter of the elongated tube 61 by suitably utilizing the space beside the collection channel 72.

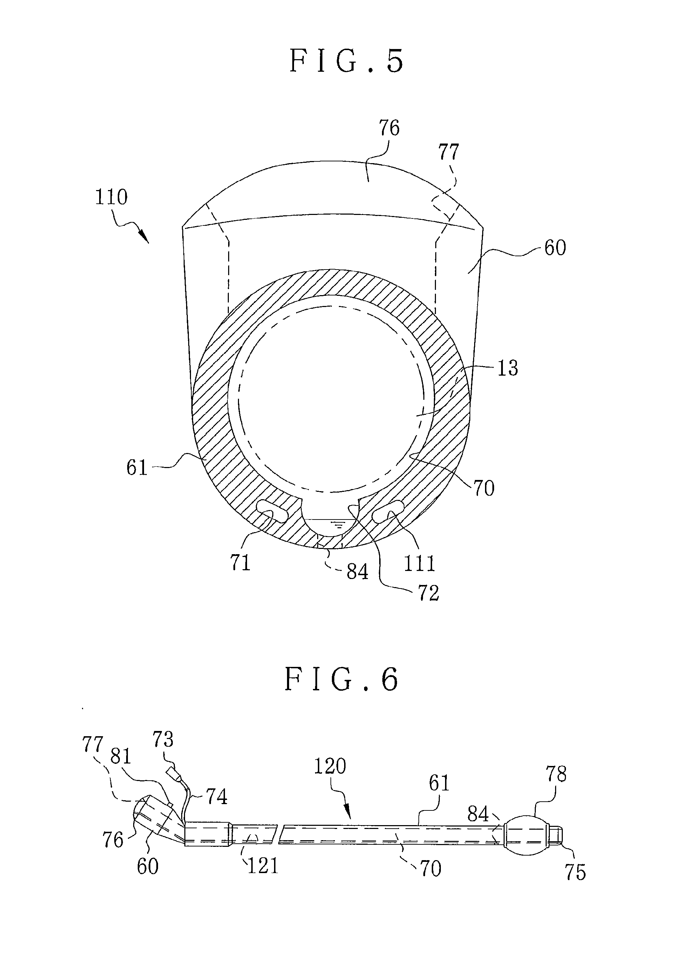

In Fig. 5, a balloon tube assembly 110 or overtube assembly or outer tube assembly as guide device is illustrated. The inflation lumen 71 is disposed in a tube wall beside the collection channel 72. Elements similar to those of the above embodiments are designated with identical reference numerals. It is also possible to dispose an additional lumen 111 on a side opposite to the inflation lumen 71 for other functions, such as supply of air or water.

It is also possible to add a structure for suction of body fluid or mucus collected in the collection channel. For example, a suction opening can be formed in the proximal end handle 60 for communicating with the collection channel. A suction apparatus is connected to the suction opening to discharge the body fluid.

In Fig. 6, another preferred balloon tube assembly 120 or overtube assembly or outer tube assembly as guide device is illustrated. A collection channel 121 for fluid extends through a distal end of the elongated tube 61 to a proximal end of the proximal end handle 60. In a manner similar to the forceps channel in the endoscope, the collection channel 121 can be operated so that a treatment instrument or another endoscope of an extreme fine size separate from the endoscope 10 is entered through the collection channel 121. It is possible to observe a tube wall or states of expansion and compression of a balloon, and to perform a task of the treatment. Also, an exit opening of the collection channel can be formed directly in front of the annular groove 85 or directly behind the annular groove 86 in place of the distal end of the elongated tube 61, so as to facilitate the observation of the tube wall or states of expansion and compression of the balloon.

In the above embodiments, the endoscope system is a single balloon endoscope system in which the balloon is mounted only on the overtube. However, an endoscope system of the invention may be a double balloon endoscope system in which an inflatable balloon is additionally mounted on an insertion tube of an endoscope. For this endoscope system, the distal end portion of the insertion tube of the endoscope can be entered deeply to reach an internal site in a gastrointestinal tract. To this end, a series of operation steps are carried out. In a first step, the insertion tube of the endoscope is entered. In a second step, the balloon of the distal end portion is expanded to anchor the insertion tube in the body cavity. In a third step, the overtube is entered to advance by following the insertion tube. In a fourth step, the balloon of the overtube is expanded for anchoring in a body part in the gastrointestinal tract. In a fifth step, the overtube is pulled manually. Note that a guide device for an endoscope of the invention can be a type without a balloon.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A guide device for mounting on an endoscope (10), **characterized in** comprising:
an elongated tube (61), having a tube lumen (70) for receiving entry of an insertion tube (13) of said endoscope, for entry in a body cavity together with said endoscope;
a collection channel (72, 121), formed inside said elongated tube together with said tube lumen, for collecting body fluid present between said insertion tube of said endoscope and an inner surface of said tube lumen;
wherein a profile of said elongated tube is substantially circular when viewed in a cross section, and a combination of said collection channel and said tube lumen within said elongated tube is substantially in a keyhole shape when viewed in a cross section.

2. A guide device as defined in claim 1, **characterized in** further comprising a proximal opening, positioned at a proximal end of said tube lumen, and adapted to exhaust of air from said collection channel.

3. A guide device as defined in claim 2, **characterized in that** said proximal opening initially receives entry of said insertion tube, and openness of said proximal opening increases outwards.

4. A guide device as defined in any one of claims 1-3, **characterized in** further comprising a fluid opening, formed in an outer surface of said elongated tube at said collection channel, for intake of said body fluid.

5. A guide device as defined in any one of claims 1-4, **characterized in** further comprising at least one through lumen formed in a wall of said elongated tube.

6. A guide device as defined in claim 5, **characterized in that** said at least one through lumen is constituted by two lumens between which said collection channel is disposed.

7. A guide device as defined in any one of claims 1-6, **characterized in that** said collection channel extends substantially at a full length of said elongated tube, and is used for receiving entry of a small second endoscope or treatment instrument.

8. A guide device as defined in any one of claims 1-7, **characterized in** further comprising a proximal end tube for constituting one end of said elongated tube;
wherein said collection channel is at least disposed adjacently to a distal side of said proximal end tube.

9. A guide device as defined in any one of claims 1-8, **characterized in** further comprising a balloon provided at a distal end of said elongated tube.

10. A guide device as defined in claim 9, **characterized in** further comprising an inflation opening, formed in said elongated tube, disposed within said balloon;
wherein said through lumen communicates with said inflation opening, and expands and compresses said balloon with air.

11. A guide device as defined in claim 9 or 10, **characterized in that** said guide device is a balloon tube assembly.
